# EUROPÄISCHE PATENTSCHRIFT

(11) **EP 3 062 762 B1**
(45) Veröffentlichungstag und Bekanntmachung des Hinweises auf die Patenterteilung: **11.12.2019**
(21) Anmeldenummer: 14790023.7
(22) Anmeldetag: 10.10.2014
(51) Int. Cl.: A61K 8/34, A61K 8/42, A61Q 13/00, A61Q 19/00, A61Q 19/10, A61K 47/10, A61K 9/08, A61K 31/045, A61K 31/16

(54) **STABILE LÖSUNGEN MIT PHYSIOLOGISCHEM KÜHLREAGENZ UND DEREN VERWENDUNG**
STABLE SOLUTIONS CONTAINING A PHYSIOLOGICAL COOLING REAGENT AND USE THEREOF
SOLUTIONS STABLES COMPRENANT UN RÉACTIF DE REFROIDISSEMENT PHYSIOLOGIQUE ET UTILISATION DESDITES SOLUTIONS

(30) Priorität: 01.11.2013 DE 102013112065
(43) Veröffentlichungstag der Anmeldung: 07.09.2016
(73) Patentinhaber: Minasolve SAS, 59310 Beuvry-la-Forêt (FR)
(72) Erfinder: NAHRWOLD, Marcus, 32423 Minden (DE); MOMMÉJA, Anna, FR-76420 Bihorel (FR)
(74) Vertreter: Brantsandpatents bvba
(86) Internationale Anmeldenummer: PCT/EP2014/071737
(87) Internationale Veröffentlichungsnummer: WO 2015/062844

(56) Entgegenhaltungen:
- EP-A1- 1 496 095
- EP-A1- 1 743 880
- EP-A1- 2 014 273
- DE-A1-102006 047 155
- DE-A1-102008 043 586
- US-A- 5 891 427
- US-A1- 2011 305 657
- US-A1- 2012 053 152
- US-A1- 2012 213 717

## Beschreibung

Diese Erfindung betrifft stabile Lösungen einzelner physiologischer Kühlreagenzien. Als Lösungsmittel dient mindestens ein mindestens zweiwertiger Alkohol. Zur Stabilisierung der Lösungen in der Kälte und zur Absenkung ihrer Viskosität ist Wasser zugesetzt.

### Beschreibung des Standes der Technik

Physiologische Kühlreagenzien finden breite Anwendung in zahlreichen Produkten, unter anderem in Kosmetika und Waschmitteln (z. B. in Zahnpasten, Rasierschäumen, Aftershaves, Deodorants, Duschgels, kühlenden Cremes und Lotionen), in Riechstoffen (z. B. in Parfümen, Raumsprays), sowie in Nahrungs- und Genussmitteln (z. B. in Kaugummis, Erfrischungsgetränken, Tabakwaren). Grundlage des physiologischen Effektes von Kühlreagenzien ist ihre Wechselwirkung mit in der Haut und in Schleimhäuten eingebetteten Nervenzellen. Die Bindung des Kühlreagenzes an einen Kälterezeptor bewirkt eine Öffnung von Ca²⁺-Ionenkanälen. Der dadurch ausgelöste Zustrom von Ca²⁺-Ionen in die Nervenzellen erzeugt den gleichen Nervenreiz, der auch durch physikalische Einwirkung von Kälte induziert wird (Nature 2002, 416, 52-58).

Das bekannteste physiologische Kühlreagenz ist L-Menthol. Typische Eigenschaften von L-Menthol sind sein campherartiger Geruch und sein bitterer, minzeartiger Geschmack. Falls eine physiologische Kühlwirkung ohne gleichzeitige Aromatisierung angestrebt wird, muss auf alternative Kühlreagenzien zurückgegriffen werden. Diese unterscheiden sich von L-Menthol in Geruch und Geschmack, sowie in der Dauer, der Intensität und der Zeitspanne bis zum Eintritt ihrer Kühlwirkung. Zur Optimierung des Kühleffektes werden häufig mehrere Kühlreagenzien miteinander kombiniert.

Viele physiologische Kühlreagenzien sind fettlösliche Substanzen, die in Wasser nur schwer löslich sind (z. B. L-Menthol: 0,355 g/L bei 20 °C, Zeitschrift für Physikalische Chemie 1930, 150, 370-371; WS-23: ca. 0,5 g/L bei 20 °C, Sicherheitsdatenblatt der Firma Renessenz). Diese Eigenschaft erschwert ihre Anwendung in wasserhaltigen Zubereitungen.

Als Reinstoff liegt die Mehrheit der physiologischen Kühlreagenzien unter Standardbedingungen (p= 1,013 bar und T=25°C) als pulverförmiger Feststoff oder als erstarrte Schmelze vor. Nachteilig bei der Handhabung derartiger Materialien ist, dass Stäube aus Pulvern ebenso wie Dämpfe aus heißen Schmelzen die Schleimhäute reizen. Daher erfordert die Anwendung fester physiologischer Kühlreagenzien im industriellen Maßstab den Einsatz geeigneter Maßnahmen zum Arbeitsschutz, wie beispielsweise Atemschutzmasken, Staubschutzmasken in Kombination mit Korbbrillen, lokale Absaugungen oder Vergleichbares. Eine Kontamination des Arbeitsplatzes kann häufig nur mit besonderen Vorrichtungen vermieden werden. Dieser Umstand erschwert und verteuert die technische Anwendung fester Kühlreagenzien.

Folgende Möglichkeiten zur Verwendung fester, lipophiler Kühlreagenzien in hydrophilen, insbesondere wässrigen Zubereitungen sind bekannt:
- Durch **Schmelzen** der Kühlreagenzien bei erhöhter Temperatur während des Mischvorgangs wird eine homogene Einarbeitung in das Endprodukt gewährleistet. Dies erfordert den Einsatz von Energie, sowie ggf. die risikovolle Handhabung heißer Schmelzen. Während der Abkühlung kann es zudem zur erneuten Kristallisation des Kühlreagenzes kommen. Diese Schwierigkeit kann insbesondere bei der Einarbeitung in wässrige Zubereitungen auftreten.
- Zur besseren Mischbarkeit mit Wasser und zur Stabilisierung der Kühlreagenzien in wässriger Lösung werden Hilfsstoffe, insbesondere Lösemittel, eingesetzt. Diese Lösemittel sollen eine Auskristallisation oder ein Ausölen des lipophilen Kühlreagenzes im wasserhaltigen/ wasserbasierten Produkt verhindern. Ein besonders geeignetes stabilisierendes **Lösungsmittel** ist **Ethanol.** Allerdings ist Ethanol eine flüchtige, reizende und leichtentzündliche Substanz und daher nur mit Produkten kompatibel, die in dauerhaft geschlossenen Gefäßen aufbewahrt werden, beispielsweise in Spraydosen, Deorollern oder Airless dispensers. Andernfalls würde die Produktqualität durch Verdunstung des Lösungsmittels verändert. Zudem stellt die Entwicklung leichtentzündlicher Ethanoldämpfe ein sicherheitstechnisches Risiko während der Produktion und bei der Handhabung entsprechender Produkte dar. Ein zusätzlicher Nachteil ist die hautreizende, hautpenetrierende, Hautfett lösende und Haut austrocknende Wirkung von Ethanol. Darüber hinaus ist Ethanol als physiologisches Wärmereagenz bekannt und kann somit der physiologischen Kühlwirkung von Kühlreagenzien entgegenwirken.
- Gemäß mehrerer Publikationen (z. B.: US2007231278A1, US2012053152A1, EP2457555A2) können Kühlreagenzien, wie beispielsweise L-Menthol oder WS-23, in den kurzkettigen mehrwertigen Alkoholen **1,2-Propandiol, 1,3-Propandiol oder Glycerin** aufgenommen und anschließend in wässrige Mischungen eingetragen werden. Eigene Versuche haben ergeben, dass Wasser bei diesen drei Lösungsmitteln als effektives Anti-Solvens für lipophile Kühlreagenzien wirkt. Darüber hinaus wird die Anwendung der Konzentrate in lipophilen Gemischen durch den hydrophilen Charakter dieser drei Lösungsmittel erschwert. So beträgt der dekadische Logarithmus des n-Oktanol-Wasser-Verteilungskoeffizienten log *K*_{ow} dieser Stoffe -0,90 für 1,2-Propandiol, -1,04 für 1,3-Propandiol und -1,76 für Glycerin. Es kommt daher aufgrund des hydrophilen Charakters der drei Stoffe zum "Ausölen" der Konzentrate in Neutralöl und insbesondere in Paraffinöl. Weiterhin sind Lösungen von beispielsweise L-Menthol oder WS-23 in den genannten drei kurzkettigen mehrwertigen Alkoholen bei niedrigen Temperaturen nicht stabil. Daher müssen die entsprechenden konzentrierten Lösungen jeweils frisch aus den Einzelkomponenten gemischt werden. Somit kann eine Handhabung der reizenden Feststoffe nicht vermieden werden.
   Der Patentantrag US2011305647 der Fa. Symrise beschreibt die synergistische Wirkung von Mischungen aus Polyolen, insbesondere **1,2-Pentandiol** und Menthylethylamidooxalat (Frescolat X-Cool). Allerdings wurden die beiden einzelnen Komponenten lediglich separat in Zubereitungen eingesetzt. Mögliche vorteilhafte Eigenschaften entsprechender vorgefertigter Konzentrate werden nicht erwähnt.
   Eine Mischung aus **1,3-Butandiol** (Butylenglycol), Octyldodecylpyroglutamat (Octyldodecyl PCA) und Menthyl PCA wird von der Firma Solabia unter dem Handelsnamen Cryogenyl vertrieben. In diesem Gemisch aus drei Substanzen hat das Kühlreagenz Menthyl PCA jedoch den geringsten Massenanteil. Dementsprechend ist die Verwendung größerer Mengen dieses Produkts zur Erzielung eines Kühleffektes erforderlich. Zur Stabilisierung der Lösung des lipophilen Mentholesters Menthyl PCA in dem hydrophilen Lösungsmittel 1,3-Butandiol (log Kow = -0,728) ist der Zusatz des lipophilen Esters Octyldodecylpyroglutamat erforderlich. Dadurch ist die Lösung nicht mit Wasser mischbar.
- **Niedrigschmelzende eutektische Mischungen** aus mehreren unterschiedlichen physiologischen Kühlreagenzien sind unter Normalbedingungen flüssig. Viele derartige Eutektika sind jedoch bei niedrigen Temperaturen hochviskose Öle, wodurch eine genaue Dosierung ebenso erschwert wird, wie eine vollständige Restentleerung der Gebinde. Außerdem schränkt die festgelegte Zusammensetzung der Mischungen die Auswahl des Anwenders ein. So entfällt die Möglichkeit, den gewünschten Kühleffekt durch Zugabe unterschiedlicher relativer Mengen der einzelnen Komponenten flexibel und individuell einzustellen.

### Zielsetzung der Erfindung:

Die Aufgabe bestand darin, konzentrierte Lösungen einzelner physiologischer Kühlreagenzien zu erhalten, die mit lipophilen und/oder hydrophilen Zubereitungen mischbar sind. Die Lösungen sollen auch bei tiefer Temperatur leicht dosierbar sein. Die Zusammensetzung oder der Aggregatzustand soll in einem weiten Temperaturbereich unverändert bleiben. Die Konzentrate sollen eine individuelle Dosierung der einzelnen Kühlreagenzien in flüssiger Form ermöglichen, um so den gewünschten sensorischen Effekt einzustellen. Die Einarbeitung der Konzentrate in Zubereitungen soll ohne Erhitzen möglich sein, d.h. bei Temperaturen von 15-25 °C.

### Detaillierte Beschreibung der Erfindung:

Diese Aufgabe wurde überraschenderweise gelöst mit stabilen Lösungen, umfassend
a) zu 10-70 Gew.-% ein physiologisches Kühlreagenz
   a1) aus der Gruppe der N-alkylierten Carboxamide der allgemeinen Formel (I) 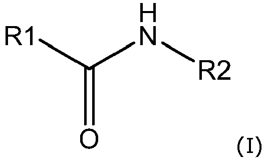 wobei
      - R1: ein Rest mit 6 bis 12 Kohlenstoffatomen ausgewählt unter verzweigten Alkylresten und Cycloalkyl mit mindestens einem weiteren C1-C3-Alkylsubstituenten am Ring ist; und
      - R2: ein Rest mit 1 bis 10 Kohlenstoffatomen ausgewählt aus verzweigtem oder unverzweigtem C1-C6-Alkyl, C3-C6-Cycloalkyl und Phenyl ist; wobei R2 einen weiteren Substituenten ausgewählt unter Heteroaryl, -CO₂-C1-C3-Alkyl, -CN, -OH, -H, C1-C3-Alkyl-C(=O)-NH2 und -O-C1-C3-Alkyl aufweist,
      oder
   a2) mit cyclischer Monoterpenstruktur der allgemeinen Formel (II) 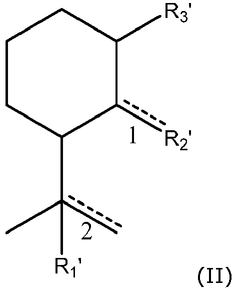 wobei
      - -----1, 2: jeweils unabhängig voneinander Einfach- oder Doppelbindungen sind,
      - R1': kein Substituent (--- 2 Doppelbindung), Wasserstoff oder -OH (--- 2 Einfachbindung) ist,
      - R2': -OH (--- 1 Einfachbindung), =0 (--- 1 Doppelbindung), -COOH (--- 1 Einfachbindung), -COOCH₂-CH(OH)-CH₂-OH (--- 1 Einfachbindung), -OC(=O)R4' (--- 1 Einfachbindung), -O-R5' (--- 1 Einfachbindung) ist oder mit R3' eine Struktur bestehend aus einem oder mehreren konsierten Ringen bildet, welche mindestens einen weiteren Substituenten -OH und/oder CH₃ trägt (z.B. Cubebol), R3' Wasserstoff ist oder mit R2' die oben beschriebene Struktur aus einem oder mehreren kondensierten Ringen bildet, und
      - R4', R5': ein C1-C5-Alkyl- oder Cylcoalkylrest ist, welcher mindestens ein weiteres Heteroatom ausgewählt aus Sauerstoff und Stickstoff enthält oder in Form (mindestens) eines Substituenten aufweist,
   und
b) zu 90-30 Gew.-% mindestens einen mindestens zweiwertigen Alkohol gemäß der allgemeinen Formel (III) enthält 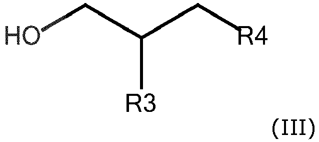 wobei
   - R3: Wasserstoff oder eine -OH-Gruppe ist und
   - R4: ausgewählt ist aus -C2-C6-Alkyl, -C2-C6-Alkyl mit OH-Gruppe in variabler Position, -O-C2-C8-Alkyl und -O-C(=O)-C2-C10-Alkyl,
   wobei mindestens einer der Reste R3, R4 eine OH-Gruppe ist oder aufweist,
   und
c) zu 0,1-25 Gew.-% Wasser enthält,
   wobei sich die Gewichtsprozente von (a), (b) und (c) zu 100 addieren.

In bevorzugten Ausführungsformen besteht die erfindungsgemäße stabile Lösung aus den Komponenten (a), (b) und (c) d.h. es sind keine weiteren Bestandteile vorhanden.

Das "physiologische Kühlreagenz (a) aus der Gruppe der *N*-alkylierten Carboxamide der allgemeinen Formel (I) (a1) oder aus der Gruppe von Substanzen mit cyclischer Monoterpenstruktur der allgemeinen Formel (II) (a2)" wird nachfolgend synonym auch verkürzt als "physiologisches Kühlreagenz (a)" oder kurz als "(a)" bezeichnet. "(a)" kann sein (a1) oder (a2) oder eine strukturelle Mischung aus (a1) und (a2). Der "mindestens eine mindestens zweiwertige Alkohol (b) gemäß der allgemeinen Formel (III)" wird nachfolgend synonym auch verkürzt als "mehrwertiger Alkohol (b)" oder kurz als "(b)" bezeichnet.

Der Begriff "Mischung" kann synonym auch als Lösung des physiologischen Kühlreagenz (a) im mehrwertigen Alkohol (b) verstanden werden. Insbesondere ist hierunter eine stabile Lösung des physiologischen Kühlreagenz (a) im mehrwertigen Alkohol (b) zu verstehen, welche bei vorzugsweise 0°C bis +25°C stabil ist. "stabil" bedeutet, dass der Aggregatzustand (=flüssig, umfassend ein und mehrphasige System) sich über diesen Temperaturbereich nicht ändert und/oder die Viskosität der Lösung über diesen Temperaturbereich im Bereich zwischen 10 und 400 mPas bleibt. Ggf. umfasst diese Lösung auch wie nachfolgend im Detail erläutert Wasser (c) und weitere mehrwertige Alkohole (b').

Der Rest R2 des physiologischen Kühlreagenz aus der Gruppe der N-alkylierten Carboxamide (a1) weist vorzugsweise mindestens einen weiteren Substituenten auf, welcher ausgewählt ist aus Heteroaryl, -CO₂-C1-C3-Alkyl, -CN, -OH, C1-C3-Alkyl-C(=O)-NH₂ und -O-C1-C3-Alkyl.

Besonders bevorzugt als physiologisches Kühlreagenz der allgemeinen Formel (I) (a1) sind Substanzen mit R1 = verzweigter C1-C6-Alkylrest, wobei WS-23 (2-Isopropyl-N,2,3-trimethylbutanamid) besonders bevorzugter Vertreter des physiologischen Kühlreagenz (a1) ist.

Hinsichtlich des physiologischen Kühlreagenz' (a2) gemäß der allgemeinen Formel (II) sind bevorzugte Vertreter die einleitend zu den p-Methan-Alkoholen und p-Methan-Ketonen, sowie die zu den O-funktionalisierten p-Methanderivaten und den C-funktionalisierten Menthanderivaten aufgelisteten bzw. abgebildeten Substanzen. Stärker bevorzugt sind als physiologisches Kühlreagenz der allgemeinen Formel (II) (a2) Menthol (R2': -OH, --- 1 Einfachbindung, --- 2 Einfachbindung, R1': Wasserstoff), Mentholester (R2':-OC(=O)R4') und-ester (R2': -O-R4'), sowie Menthon-Derivate (R2' gleich =O). Der Rest R4' ist hierbei vorzugsweise ausgewählt aus -C(=O)-CH(OH)-CH₃, -C(=O)-CH₃, PCA, C(=O)-C(=O)-NH-C₂H₅, -C(=O)-C₂H₄-COOH, - C(=O)-C₃H₆-COOH, -C(=O)-C₂H₄-C(=O)-N(CH₃)₂, -C(=O)-O-C₂H₄-OH, der Rest R5' ist vorzugsweise ausgewählt aus -CH₂-CH(OH)-CH₂-OH und cyclischem Acetal mit - CH₂-OH-Gruppe (z.B. Menthonglycerinacetal) und cyclischem Acetal mit Keto- und CH₃-Gruppe (z.B. FEMA 4285). L-Menthol ist der höchst bevorzugte Vertreter von (a2).

Der mindestens eine, mindestens zweiwertige Alkohol (b) gemäß der allgemeinen Formel (II) hat vorzugsweise einen log Kow-Wert von ≥ -0,85. Insbesondere hat er einen log Kow-Wert zwischen -0,85 und +2,5.

In bevorzugter Ausführungsform der stabilen Lösung sind neben dem physiologischen Kühlreagenz (a) mindestens zwei mindestens zweiwertige Alkohole (b) der allgemeinen Formel (III) vorhanden. Vorzugsweise besteht die stabile Lösung in dieser Ausführungsform daher aus (a), einem mindestens zweiwertigen Alkohol (b) und einem oder mehreren weiteren mindestens zweiwertige(n) Alkohole(n) (b') der allgemeinen Formel (III), welche verschieden vom ersten Alkohol (b) sind.

Besonders bevorzugt ist der mindestens eine mindestens zweiwertige Alkohol (b) ausgewählt aus 1,2-Pentandiol, 1,5-Pentandiol, 1,2-Hexandiol, 1,6-Hexandiol, 1,2,3-Hexantriol, Dipropylenglycol, 1,2-Octandiol, Ethylhexylglycerin und Glycerylcaprylat. Falls ein oder mehrere weitere mindestens zweiwertige Alkohole (b') der allgemeinen Formel (III) vorhanden sind, erfolgt die Auswahl vorzugsweise aus derselben bevorzugten bzw. besonders bevorzugten Gruppe wie bei (b).

Der mindestens eine mindestens zweiwertige Alkohol (b) ist in einer Ausführungsform vorzugsweise ausgewählt aus zwei- oder dreiwertigen Alkoholen mit linearer oder verzweigter C5-C8-Alkylkette, d.h. in der allgemeinen Formel (III) sind die Reste R3, R4 vorzugsweise wie folgt:
R3 = H oder -OH
   und
R4 = -C2-C6-Alkyl oder -C2-C6-Alkyl mit OH-Gruppe in variabler Position -O-C2-C8-Alkyl und -O-C(=O)-C2-C10-Alkyl, wobei mindestens einer der Reste R3, R4 eine - OH-Gruppe ist oder aufweist.

Besonders bevorzugt ist der mindestens eine mindestens zweiwertige Alkohol (b) in dieser Ausführungsform ausgewählt aus 1,2-Pentandiol, 1,5-Pentandiol, 1,2-Hexandiol, 1,6-Hexandiol, 1,2,3-Hexantriol, und 1,2-Octandiol. Falls in dieser Ausführungsform ein oder mehrere weitere mindestens zweiwertige(n) Alkohole(n) b') der allgemeinen Formel III vorhanden sind, erfolgt die Auswahl vorzugsweise aus derselben besonders bevorzugten Gruppe wie bei (b).

Chirale mehrwertige Alkohole (b) bzw. (b') liegen als reines Enantiomer, als racemisches Gemisch oder als enantiomeren-angereichertes Gemisch bzw. als reines Diastereomer oder als Diastereomerengemisch vor.

Das Konzentrationsverhältnis der stabile Lösung ist vorzugsweise dadurch gekennzeichnet, dass
das physiologische Kühlreagenz (a) in 10-70 Gew.-%, vorzugsweise 40-60 Gew.-%; und
der mindestens eine mindestens zweiwertige Alkohol (b) in 90-30 Gew.-%, vorzugsweise 60-40 Gew.-% enthalten ist; und dass Wasser (c) zu 0,1-25 Gew.-%, vorzugsweise 0,1-10 Gew.-%, höchst bevorzugt 5-10 Gew.-% vorhanden ist, wobei sich die Gewichtsprozente von (a), (b) und des Wassers (c) zu 100 addieren, d.h. in anderen Worten weist diese bevorzugte Ausführungsform nur (a), (b) und (c) auf/besteht aus diesen drei Komponenten. Ggf. ist/sind weitere Alkohole (b') vorhanden, so dass die stabile Lösung aus (a), (b), (b') und (c) besteht.

Diese Angaben beziehen sich insbesondere auf stabile Lösungen bei mindestens Raumtemperatur (RT, 20°C), und auf alle mehrwertigen Alkohole (b) und ggf. (b') mit mindestens 5 Kohlenstoffatomen. Bei Alkoholen mit mehr als 5 Kohlenstoffatomen ist besonders bevorzugt (a) zu 10-40 Gew.-% und (b) (ggf. (b') zu 90-60 Gew.-% bei RT vorhanden, insbesondere bei T≥25°C.

Es konnte gezeigt werden, dass physiologische Kühlreagenzien (a), beispielsweise WS-23 (a1) oder L-Menthol (a2), gut löslich sind in Alkoholen (b) gemäß der allgemeinen Formel (III). Es konnte ebenfalls gezeigt werden, dass entsprechende konzentrierte Lösungen von (a) in (b) auch bei niedrigen Temperaturen, teilweise unterhalb von 0 °C, stabil sind. Die erfindungsgemäßen stabilen Lösungen sind stabiler als vergleichbare Lösungen der Komponente (a), beispielsweise WS-23 oder L-Menthol, in kürzerkettigen (< 5 C-Atome) mehrwertigen Alkoholen wie 1,2-Propandiol, 1,3-Propandiol oder Glycerin. Diese ergeben erst bei Konzentrationen ≥ 50 Gew.-% des mehrwertigen Alkohols und T ≥ 25 °C bzw. bei Konzentrationen ≥ 60 Gew.-% des mehrwertigen Alkohols und T ≥ 20 °C stabile einphasige Lösungen. Weitere Details zur Löslichkeit und Stabilität bei tieferen Temperaturen werden nachfolgend im experimentellen Teil vorgestellt.

Die Herstellung der stabilen Lösung erfolgt vorzugsweise so, dass die einzelnen Komponenten der stabilen Lösung bei einer Temperatur von 0 °C bis 100 °C, bevorzugt bei einer Temperatur von 25 °C bis 70 °C, besonders bevorzugt bei einer Temperatur von 35 bis 55 °C miteinander in Kontakt gebracht werden.

Nach dem Stand der Technik war davon auszugehen, dass Wasser in Kombination mit lipophilen mehrwertigen Alkoholen in Gegenwart eines physiologischen Kühlreagenz als Anti-Solvens wirken würde. Dies konnte für die kürzerkettigen (<4 C-Atome) mehrwertigen Alkohole wie 1,2-Propandiol, 1,3-Propandiol oder Glycerin erwartungsgemäß gezeigt werden.

Stattdessen wurde für die erfindungsgemäßen mehrwertigen Alkohole (b) aber gefunden, dass die Zugabe geringer Wassermengen die Stabilität der stabilen Lösungen bei niedrigen Temperaturen zum Teil sogar erhöht (vgl. experimenteller Teil, Tabelle 2, Einträge 2, 6, 8 und 10).

Die wasserhaltigen Lösungen des physiologischen Kühlreagenz (a) weisen zum großen Teil sogar tiefere Kristallisations- und Schmelztemperaturen auf, als die entsprechenden wasserfreien Lösungen. Dies gilt insbesondere für Lösungen mit einem hohen Gehalt an physiologischem Kühlreagenz (a). Dieser Befund war insofern nicht zu erwarten, als in der Literatur beschriebene Lösungen von beispielsweise WS-23 in 1,2-Propandiol oder Glycerin bereits bei Zugabe einer geringen Menge Wasser auskristallisieren. Bemerkenswert ist insbesondere, dass weder WS-23, noch 1,2-Octandiol oder Ethylhexylglycerin mit Wasser mischbar sind. Dennoch bleiben Lösungen des physiologischen Kühlreagenz (a), beispielsweise von WS-23, in 1,2-Octandiol oder Ethylhexylglycerin auch nach Zugabe von 10 % Wasser homogen und klar. Im Falle von 1,2-Octandiol wird zudem eine deutliche Schmelzpunkterniedrigung der ternären Mischung beobachtet. Dieser synergistische Effekt (vgl. experimenteller Teil, Tabelle 2, Eintrag 8) war insofern überraschend, als die beiden Einzelkomponenten WS-23 (FP 55 °C) und 1,2-Octandiol (FP 35 °C) Schmelzpunkte deutlich oberhalb von 25 °C aufweisen und auch als Mischung bei 20 °C als Feststoff vorliegen (siehe experimenteller Teil, Tabelle 2, Eintrag 7).

Die Ermittlung der Schmelz- und Erstarrungstemperatur zeigte, dass die erfindungsgemäßen stabilen Lösungen in Kombination mit Wasser überraschend kältestabil sind, d.h. auch bei Temperaturen unterhalb von 0 °C noch stabil in flüssiger, nicht entmischter kristallfreier Form vorliegen. Nach Vermengung mit Wasser oder mehrphasigen wasserhaltigen Gemischen tritt in einem weiten Temperaturbereich keine Auskristallisation einer der Komponenten ein. Dies gilt insbesondere für die stabilen Lösungen aus physiologischem Kühlreagenz (a), mehrwertigem Alkohol (b) ausgewählt aus zwei- oder dreiwertigem Alkohol mit linearer oder verzweigter C5-C8-Alkylkette, und Wasser (c).

Kältestabile Lösungen wie die erfindungsgemäßen stabilen Lösungen sind vorteilhaft, da sie auch bei Lagerung in kalter Umgebung ihre Zusammensetzung beibehalten. Sie müssen daher während eines Transports nicht beheizt werden. Auch unmittelbar vor ihrer Anwendung müssen die Lösungen nicht erwärmt und homogenisiert werden, wodurch Zeit, Energie und Produktionskapazität eingespart werden. Dies ist ein Vorteil gegenüber vielen gegenwärtig bekannten flüssigen Kühlreagenzien, die in der Regel einen deutlich höheren Schmelzpunkt aufweisen. Flüssige stabile Lösungen erlauben generell eine einfache und genaue Dosierung - nach Bedarf auch kontinuierlich durch Zupumpen oder Eingießen. Die Einarbeitung in flüssige Produkte ist schneller, effektiver und gleichmäßiger als die Einarbeitung der Kühlreagenzien als Feststoff. Beispielsweise wird eine Klumpenbildung vermieden. Flüssige Konzentrate ermöglichen außerdem durch Vermeidung von Stäuben eine sicherere Handhabung der haut-, schleimhaut- und augenreizenden Substanz.

Weiterhin wurde gefunden, dass die Viskosität der erfindungsgemäßen stabilen Lösungen aus physiologischem Kühlreagenz (a), Alkohol (b) und Wasser (c) gering ist, d.h. zumindest bei T zwischen 10 und 30°C unter 400 mPas liegt. Sie kann zudem durch Zugabe von Wasser in einem weiten Temperaturbereich deutlich, d.h. um mindestens 40 mPas, unter die Viskosität der wasserfreien Mischung herabgesetzt werden. Durch eine niedrigere Viskosität werden sowohl die genaue Dosierung der Lösung, wie auch eine vollständige Restentleerung der Gebinde deutlich erleichtert.

Weiterhin neuartig ist die gleichzeitig erhalten bleibende gute Mischbarkeit der wasserhaltigen Konzentrate mit lipophilen kosmetischen Lösemitteln. Demgegenüber sind an literaturbekannte Formulierungen angelehnte Lösungen von WS-23 in Ethanol/Wasser, Glycerin/Wasser oder Propandiol/Wasser nicht mit Fetten oder Ölen mischbar. Bei den erfindungsgemäßen stabilen Lösungen tritt auch nach Vermengung mit lipophilen Phasen (z.B. Neutralöl, pflanzliche Öle, sonstige Fettsäureester, Paraffine bzw. Paraffinöle) oder mehrphasigen ölhaltigen Gemischen keine Auskristallisation einer der Komponenten ein.

Auf Grundlage der gewonnenen neuen Erkenntnisse ist davon auszugehen, dass Lösungen von physiologischem Kühlreagenz (a), beispielsweise WS-23 (a1) oder L-Menthol (a2) in mehrwertigen Alkoholen (b) und Wasser (c) einen gegenüber bereits bekannten Mischungen erweiterten Anwendungsbereich aufweisen. Insbesondere wird dies deutlich bei der Anwendung der erfindungsgemäßen stabilen Lösungen als Einsatzstoffe für klare Hydrogele mit hautkühlender Wirkung.
Erfindungsgemäß ist daher auch die Verwendung der oben dargelegten stabilen Lösungen für kosmetische und/oder pharmazeutische und/oder dermatologische und/oder hygienische und/oder Lebensmittel-relevante Zubereitungen.

Ebenso ist erfindungsgemäß ein Produkt, insbesondere ein kosmetisches und/oder pharmazeutisches und/oder dermatologisches und/oder hygienisches und/oder Lebensmittel-relevantes Produkt, enthaltend eine stabile Lösung wie oben erläutert.

Als "hygienische Zubereitung" bzw. "hygienisches Produkt" werden insbesondere Haushalts- oder Reinigungsprodukte verstanden. Als "Lebensmittel-relevante Zubereitung" bzw. "Lebensmittel-relevantes Produkt" werden insbesondere der Ernährung oder dem Genuss dienende Zubereitung, sowie Riechstoff- oder Geschmacksstoffzubereitung verstanden.

Das jeweilige Produkt kann in beliebiger Form vorliegen, insbesondere als:
a. Lösung,
b. Suspension,
c. Emulsion,
d. Gel,
e. Salbe,
f. Paste,
g. Pulver,
h. in Stücken oder als Block vorliegender Feststoff,
i. Schaum,
j. auf Mikroverkapselung, Liposomen oder ähnlichen mikroskopischen Strukturen basierendes Formulierungssystem.

Ohne Bindung an diese Theorie ist anzunehmen, dass die Komponenten der flüssigen stabilen Lösung abgesehen von (a) als Lösevermittler zwischen dem darin enthaltenen physiologischen Kühlreagenz (a) und den übrigen Komponenten des Produkts wirken.

Die im Produkt enthaltenen mehrwertigen Alkohole (b) oder ggf. (b') können eine zusätzliche Funktion innerhalb des Fertigproduktes haben (z. B. als Feuchthaltemittel, penetrationsförderndes Reagenz, Konservierungsmittel, Verstärker der physiologischen Kühlwirkung).

Ebenso kann das im Produkt enthaltene Kühlreagenz neben einer physiologischen Kühlwirkung auch als Verstärker für andere sensorische Effekte dienen (z. B. als Geschmacksverstärker oder als Verstärker eines physiologischen Wärmeffekts).

Die Erfindung wird nachstehend anhand von Beispielen weiter erläutert, ohne sie auf diese zu beschränken.

### Beispiele / experimenteller Teil

Als physiologisches Kühlreagenz (a) werden exemplarisch WS-23 (a1) bzw. L-Menthol (a2) beschrieben/genutzt.

### Beispiel 1 - Herstellung der Lösungen

Mischungen von WS-23 bzw. L-Menthol (a) und den mindestens zweiwertigen Alkoholen (b) wurden vorteilhaft bei einer Temperatur oberhalb der Schmelzpunkte der beiden Komponenten erzeugt. Es war günstig, zunächst den mehrwertigen Alkohol (b) als flüssige Komponente vorzulegen und anschließend (a) als feste Komponente zuzudosieren. Nach Bedarf kann zu jedem Zeitpunkt eine zur Einstellung der gewünschten Viskosität erforderliche Menge Wasser zur Mischung hinzugefügt werden. Ebenfalls nach Bedarf können weitere Komponenten in geringer Menge (≤ 5 Gew.-%) zudosiert werden, die zur Stabilisierung der Mischung beitragen, beispielsweise Puffersubstanzen, Emulgatoren oder Antioxidationsmittel, wobei die Summe aller Komponenten 100 Gew.-% ergibt. Nachfolgend wird "Gew.-%" oftmals als "%" verkürzt.

"INCI" steht für Internationale Nomenklatur für kosmetische Inhaltsstoffe (Abkürzung INCI vom engl. International Nomenclature of Cosmetic Ingredients). Die entsprechenden Komponenten werden in dieser Rubrik nachfolgend in englischer Sprache genannt.

Die Ergebnisse der Mischungen aus WS-23 bzw. L-Menthol und den jeweiligen mehrwertigen Alkoholen (b) sind in Tabelle 1 gezeigt.

Die Ergebnisse zeigen, dass (a) gut löslich ist in mehrwertigen Alkoholen (b), insbesondere in zwei- oder dreiwertigen Alkoholen mit linearer oder verzweigter C5-C8-Kohlenstoffkette (Tabelle 1). Es konnte ebenfalls gezeigt werden, dass entsprechende konzentrierte Lösungen von (a) auch bei niedrigen Temperaturen stabil sind. Die erfindungsgemäßen Mischungen sind stabiler als vergleichbare Lösungen von (a) in kürzerkettigen mehrwertigen Alkoholen wie 1,2-Propandiol, 1,3-Propandiol oder Glycerin (vgl. Einträge 14-17 in Tabelle 1).

**Tabelle 1 : Aggregatzustände von Lösungen physiologischer Kühlreagenzien in mehrwertigen Alkoholen (+ = klare Flüssigkeit, 0 = Zweiphasengemisch, - = Feststoff) :**

| **Nr.** | **Mischung** | | **-5 °C** | **0 °C** | **5 °C** | **10 °C** | **15 °C** | **20 °C** | **25 °C** |
|---|---|---|---|---|---|---|---|---|---|
| **1** | WS-23 (50 %) | 1,2-Pentandiol (50 %) | 0 | 0 | 0 | 0 | 0/+ | + | + |
| **2** | WS-23 (40 %) | 1,2-Pentandiol (60 %) | 0 | 0 | 0 | + | + | + | + |
| **3** | WS-23 (30 %) | 1,2-Pentandiol (70 %) | + | + | + | + | + | + | + |
| **4** | WS-23 (40 %) | 1,5-Pentandiol (60 %) | + | + | + | + | + | + | + |
| **5** | WS-23 (50 %) | 1,2-Hexandiol (50 %) | 0 | 0 | 0 | 0 | 0 | + | + |
| **6** | WS-23 (40 %) | 1,2-Hexandiol (60 %) | + | + | + | + | + | + | + |
| **7** | WS-23 (30 %) | 1,2-Hexandiol (70 %) | + | + | + | + | + | + | + |
| **8** | WS-23 (50 %) | 1,2-Octandiol (50 %) | 0 | 0 | 0 | 0 | 0/+ | 0/+ | + |
| **9** | WS-23 (40 %) | 1,2-Octandiol (60 %) | 0 | 0 | 0 | 0 | 0/+ | + | + |
| **10** | WS-23 (40 %) | Ethylhexylglycerin (60%) | + | + | + | + | + | + | + |
| **11** | L-Menthol (50 %) | 1,2-Pentandiol (50 %) | - | 0 | + | + | + | + | + |
| **12** | L-Menthol (40 %) | 1,2-Pentandiol (60 %) | + | + | + | + | + | + | + |
| **13** | L-Menthol (40 %) | Ethylhexylglycerin (60%) | + | + | + | + | + | + | + |

| ***Nicht erfindungsgemäße Mischungen:*** | | | | | | | | | |
|---|---|---|---|---|---|---|---|---|---|
| **14** | WS-23 (40 %) | *Glycerin* (60 %) | - | - | - | - | - | - | - |
| **15** | WS-23 (50 %) | 1,2-Propandiol (50 %) | - | - | - | 0 | 0 | 0 | 0/+ |
| **16** | WS-23 (40 %) | 1,2-Propandiol (60 %) | - | - | - | 0 | 0 | 0/+ | + |
| **17** | L-Menthol (50 %) | 1,2-Propandiol (50 %) | - | - | 0 | 0 | + | + | + |

### Beispiel 2 - Zugabe von Wasser und Bestimmung von Schmelz- und Erstarrungstemperaturen

Mischungen zu je 10 g wurden aus 40 Gew.-% Kühlreagenz (a) und Alkohol (b) sowie ggf. Wasser (c) erzeugt.

Nach dem Stand der Technik war davon auszugehen, dass Wasser in Kombination mit lipophilen mehrwertigen Alkoholen als Anti-Solvens wirken würde. Stattdessen wurde jedoch gefunden, dass die Zugabe geringer Wassermengen die Stabilität der Mischungen bei niedrigen Temperaturen zum Teil sogar erhöht (vgl. Tabelle 2, Einträge 2, 6, 8 und 10). Die wasserhaltigen Lösungen weisen zum großen Teil sogar tiefere Kristallisations- und Schmelztemperaturen auf, als die entsprechenden wasserfreien Lösungen. Dies gilt insbesondere für Lösungen mit einem hohen Gehalt an WS-23. Dieser Befund war insofern nicht zu erwarten, als literaturbekannte Lösungen von WS-23 in 1,2-Propandiol oder Glycerin bereits bei Zugabe einer geringen Menge Wasser auskristallisieren. Bemerkenswert ist insbesondere, dass weder WS-23, noch 1,2-Octandiol oder Ethylhexylglycerin mit Wasser mischbar sind. Dennoch bleiben Lösungen von WS-23 in 1,2-Octandiol oder Ethylhexylglycerin auch nach Zugabe von 10 % Wasser homogen und klar. Im Falle von 1,2-Octandiol wird zudem eine deutliche Schmelzpunkterniedrigung der ternären Mischung beobachtet. Dieser synergistische Effekt (Tabelle 2, Eintrag 8) war insofern überraschend, als die beiden Einzelkomponenten WS-23 (FP 55 °C) und 1,2-Octandiol (FP 35 °C) Schmelzpunkte deutlich oberhalb von 25 °C aufweisen und auch als Mischung bei 20 °C als Feststoff vorliegen (siehe Tabelle 2, Eintrag 7).

**Tabelle 2: Schmelz- und Erstarrungstemperaturen 40%iger Konzentrate.**

| **Nr** . | **Mischung** | | | **Schmelztemperatur** | **Kristallisationstemperatur** |
|---|---|---|---|---|---|
| | **Kühlreagenz (a)** | **Alkohol (b)** | **Wasser (c)** | | |
| **1** | 4,0 g WS-23 | 1,2-Pentandiol: 6,0 g | - | - 18 °C | - 19 °C |
| **2** | 4,0 g WS-23 | 1,2-Pentandiol: 5,0 g | 1,0 g | < - 20 °C | < - 20 °C |
| **3** | 4,0 g WS-23 | 1,5-Pentandiol: 6,0 g | - | < - 20 °C | < - 20 °C |
| **4** | 4,0 g WS-23 | 1,5-Pentandiol: 5,0 g | 1,0 g | + 20 °C | - 20 °C |
| **5** | 4,0 g WS-23 | 1,2-Hexandiol: 6,0 g | - | - 18 °C | - 19 °C |
| **6** | 4,0 g WS-23 | 1,2-Hexandiol: 5,0 g | 1,0 g | < - 20 °C | < - 20 °C |
| **7** | 4,0 g WS-23 | 1,2-Octandiol: 6,0 g | - | > + 20 °C | > + 20 °C |
| **8** | 4,0 g WS-23 | 1,2-Octandiol: 5,0 g | 1,0 g | 8 °C | -6°C |
| **9** | 4,0 g WS-23 | Ethylhexylglycein: 6,0 g | - | > + 20 °C | > + 20 °C |
| **10** | 4,0 g WS-23 | Ethylhexylglycerin: 5,5 g | 0,5 g | + 10 °C | -4°c |
| **11** | 4,0 g L-Menthol | 1,2-Pentandiol: 6,0 g | - | - 10 °C | - 16 °C |
| **12** | 4,0 g L-Menthol | 1,2-Pentandiol: 5,0 g | 1,0 g | 0 °C | - 16 °C |

| **nicht erfindungsgemäße Mischungen:** | | | | | |
|---|---|---|---|---|---|
| **Nr** . | **Kühlreagenz (a)** | **Alkohol (b)** | **Wasser (c)** | **Schmelztemperatur** | **Kristallisationstemperatur** |
| **13** | 4,0 g WS-23 | 1,2-Propandiol: 6,0 g | - | > + 20 °C | > + 20 °C |
| **14** | 4,0 g WS-23 | 1,2-Propandiol: 5,5 g | 0,5 g | > + 20 °C | > + 20 °C |
| **15** | 4,0 g L-Menthol | 1,2-Propandiol: 5,0 g | 1,0 g | + 20 °C | 0 °C |
| **16** | 4,0 g WS-23 | 1,3-Propandiol: 6,0 g | - | > + 20 °C | > + 20 °C |
| **17** | 4,0 g WS-23 | 1,3-Propandiol: 5,0 g | 1,0 g | > + 20 °C | > + 20 °C |
| **18** | 4,0 g WS-23 | Glycerin: 6,0 g | - | > + 20 °C | > + 20 °C |
| **19** | 4,0 g WS-23 | Glycerin: 5,0 g | 1,0 g | > + 20 °C | > + 20 °C |

Zur Ermittlung der Schmelz- und Erstarrungstemperatur wurden die in Tabelle 2 aufgeführten Mischungen in 30 mL-Glasflaschen unter Rühren auf 50 °C erhitzt. Dadurch wurden klare Lösungen erhalten, die im Temperierbad auf -20 °C abgekühlt wurden. In der Kälte wurde zu jeder Mischung ein Impfkristall des Kühlreagenz gegeben. Daraufhin wurden die Probeflaschen ohne Rühren über Nacht (12-16 h) bei -20 °C im Thermostatbad gelagert. Anschließend wurden die Mischungen in 2 °C-Schritten auf 20 °C erwärmt. Nach Erreichen jeder neuen Solltemperatur wurde 30 Minuten gewartet und daraufhin der Aggregatzustand der Gemische visuell beurteilt. Die niedrigste Temperatur, bei der eine klare Lösung vorlag, wurde als "Schmelztemperatur" gewertet. Die Mischungen wurden nachfolgend über Nacht (12-16 h) bei 20 °C im Thermostatbad gelagert. Anschließend wurden die Proben analog zur beschriebenen Vorgehensweise in 2 °C-Schritten wieder auf -20 °C abgekühlt. Die höchste Temperatur, bei der eine erneute Kristallisation erfolgte, wurde als "Kristallisationstemperatur" gewertet.

### Beispiel 3 - Viskositätsbestimmung

Mischungen aus WS-23 (a1) bzw. L-Menthol (a2) und mehrwertigem Alkohol (b) wurden gemäß Beispiel 1 erzeugt und ihre Viskosität mit und ohne Wasserzusatz untersucht. Die Messungen erfolgten an einem Rotationsviskosimeter, Typ "Haake Viscotester 6 plus", mit Spindel L1 bei 20-60 U/min. Die Ergebnisse sind in Tabelle 3 wiedergegeben.

Es wurde gefunden, dass die Viskosität der erfindungsgemäßen Lösungen von Kühlreagenzien (a) in Alkoholen (b) gering ist. Sie kann zudem durch Zugabe von Wasser in einem weiten Temperaturbereich deutlich herabgesetzt werden (Tabelle 3). Durch eine niedrigere Viskosität werden sowohl die genaue Dosierung der Lösung, wie auch eine vollständige Restentleerung der Gebinde deutlich erleichtert.

**Tabelle 3: Viskositäten von Kühlreagenz-Lösungen mit und ohne Wasser.**

| **Nr.** | **Kühlreagenz(a)** | **Alkohol (b)** | **Wasser (c)** | **Viskositäten [mPas]** | | |
|---|---|---|---|---|---|---|
| | | | | **10 °C** | **20 °C** | **30 °C** |
| **1** | 40 g WS-23 | 60 g 1,2-Pentandiol | - | 137 | 137 | 96 |
| **2** | 40 g WS-23 | 50 g 1,2-Pentandiol | 10 g | 78 | 70 | 42 |
| **3** | 40 g WS-23 | 60 g 1,5-Pentandiol | - | 359 | 287 | 168 |
| **4** | 40 g WS-23 | 50 g 1,5-Pentandiol | 10 g | 166 | 130 | 91 |
| **5** | 40 g WS-23 | 60 g 1,2-Hexandiol | - | 258 | 154 | 111 |
| **6** | 40 g WS-23 | 50 g 1,2-Hexandiol | 10 g | 106 | 87 | 55 |
| **7** | 40 g WS-23 | 60 g 1,2-Octandiol | - | 280 | 231 | 128 |
| **8** | 40 g WS-23 | 50 g 1,2-Octandiol | 10 g | 106 | 106 | 62 |
| **9** | 40 g WS-23 | 60 g Ethylhexylglycerin | - | 271 | 230 | 139 |
| **10** | 40 g WS-23 | 50 g Ethylhexylglycerin | 10 g | 130 | 115 | 84 |
| **11** | 40 g L-Menthol | 60 g 1,2-Pentandiol | - | 118 | 84 | 49 |
| **12** | 40 g L-Menthol | 50 g 1,2-Pentandiol | 10 g | 58 | 41 | 20 |
| **13** | 40 g L-Menthol | 60 g 1,2-Hexandiol | - | 134 | 102 | 46 |
| **14** | 40 g L-Menthol | 50 g 1,2-Hexandiol | 10 g | 57 | 45 | 25 |

### Beispiel 4 - Mischbarkeit mit Wasser und lipophilen kosmetischen Lösemitteln

Mischungen aus 40 Gew.-% Kühlreagenz (a) und jeweils 60 bzw. 50 Gew.-% Alkohol (b) wurden gemäß Beispiel 1 erzeugt bzw. mit 10 Gew.-% Wasser versetzt.

Anschließend wurde alternativ mit Wasser, Neutralöl oder Paraffinöl versetzt. Die Ergebnisse sind in Tabelle 4 gezeigt.

**Tabelle 4: Löslichkeit von Kühlreagenzien und erfindungsgemäßen Mischungen (+ = klare Lösung, - = Zweiphasengemisch, % = Gew.-%):**

| **PKR = physiologisches Kühlreagenz** | | | | **Lagertemperatur** | **+ Wasser auf**: | | **+ Neutralöl auf:** | | | **+ Paraffinöl auf:** | |
|---|---|---|---|---|---|---|---|---|---|---|---|
| **PKR (a)** | **Alkohol (b)** | **H₂O (c)** | **Menge** | | **20 g** | **10 g** | **10 g** | **5 g** | **2,5 g** | **10 g** | **5 g** |
| WS-23 40% | 1,2-Pentandiol 60% | / | 0,25 g (= 0,1 g WS-23) | 20 °C | + | - | + | + | + | + | + |
| | | | | 8 °C | + | - | + | + | - | + | - |
| WS-23 40% | 1,2-Pentandiol 50 % | 10 % | 0,25 g (= 0,1 g WS-23) | 20 °C | + | - | + | - | - | + | - |
| | | | | 8 °C | + | - | + | - | - | - | - |
| WS-23 40% | 1,5-Pentandiol 60 % | / | 0,25 g (= 0,1 g WS-23) | 20 °C | + | + | + | + | + | + | - |
| | | | | 8 °C | + | + | + | + | + | - | - |
| WS-23 40% | 1,2-Hexandiol 60% | / | 0,25 g (= 0,1 g WS-23) | 20 °C | + | + | + | + | + | - | - |
| | | | | 8 °C | + | + | + | + | + | - | - |
| WS-23 40% | 1,2-Hexandiol 50 % | 10 % | 0,25 g (= 0,1 g WS-23) | 20 °C | + | + | + | + | - | - | - |
| | | | | 8 °C | + | + | + | - | - | - | - |
| WS-23 40% | 1,2-Octandiol 60 % | / | 0,25 g (= 0,1 g WS-23) | 20 °C | - | - | + | + | + | - | - |
| | | | | 8 °C | - | - | + | + | + | - | - |
| WS-23 40 % | 1,2-Octandiol 50 % | 10 % | 0,25 g (= 0,1 g WS-23) | 20 °C | - | - | + | + | + | + | - |
| | | | | 8 °C | - | - | + | - | - | - | - |
| WS-23 40 % | Ethylhexylglycerin 60 % | / | 0,25 g (= 0,1 g WS-23) | 20 °C | - | - | + | + | + | + | + |
| | | | | 8 °C | - | - | + | + | + | + | - |
| WS-23 40 % | Ethylhexylglycerin 50 % | 10 % | 0,25 g (= 0,1 g WS-23 | 20 °C | - | - | + | - | - | + | - |
| | | | | 8 °C | - | - | + | - | - | - | - |
| WS-23 100 % | / | / | 0,1 g | 20 °C | + | - | + | + | + | - | - |
| | | | | 8 °C | - | - | + | + | + | - | - |
| L-Menthol 40 % | 1,2-Pentandiol 60 % | / | 0,25 g (= 0,1 g Menthol) | 20 °C | - | - | + | + | + | - | - |
| | | | | 8 °C | - | - | + | + | + | - | - |
| L-Menthol 40 % | 1,2-Hexandiol 60 % | / | 0,25 g (= 0,1 g Menthol) | 20 °C | - | - | + | + | + | - | - |
| | | | | 8 °C | - | - | + | + | + | - | - |
| L-Menthol 40 % | Ethylhexylglycerin 60 % | / | 0,25 g (= 0,1 g Menthol) | 20 °C | - | - | + | + | + | + | + |
| | | | | 8 °C | - | - | + | + | + | + | + |
| L-Menthol 100 % | / | / | 0,1g | 20 °C | - | - | + | + | + | + | + |
| | | | | 8 °C | - | - | + | + | + | + | + |

### Beispielform ulierungen

### Beispiel 5 - Hautreinigungswasser mit kühlender Wirkung

| **Phase** | **Einsatzstoff** | **INCI** | % |
|---|---|---|---|
| **A** | Wasser | Aqua | ad 100 |
| | MinaCare Caprocine | Capryloyl glycine | 1,00 |
| | 10 %ige Natronlauge | Aqua (and) Sodium hydroxide | q. sat. |
| **B** | MinaCare Hexiol | 1,2-Hexanediol | 3,00 |
| | MinaCare Pentiol | Pentylene glycol | 2,00 |
| | 1,2-Butandiol | 1,2-Butanediol | 2,00 |
| **C** | Lösung von WS-23 (40 %) in 1,2-Pentandiol (50 %) und Wasser (10 %) | Pentylene Glycol (and) Methyl diisopropyl propionamide (and) Aqua | 2,00 |

Herstellung: Wasser wird auf 70-75 °C erwärmt. MinaCare Caprocine wird eingerührt (200 U/min). Der pH-Wert wird mit 10%iger Natronlauge auf pH 7,0 eingestellt und die Lösung wird unter Rühren auf 45 °C abgekühlt (Phase A). MinaCare Hexiol, MinaCare Pentiol und Butandiol werden bei 20-25 °C vermischt (Phase B). Phase B wird unter Rühren (500 U/Min) bei 45 °C zur Phase A zugegeben. Anschließend wird die Mischung wird auf 20-25 °C abgekühlt. Das Kühlreagenz-Konzentrat (Phase C) wird unter Rühren (500 rpm) zudosiert.

### Beispiel 6 - Zwei opake Hydrogele auf Polyacrylat-Basis

| **Phase** | **Einsatzstoff** | **INCI** | **%** |
|---|---|---|---|
| **A** | Wasser | Water | ad 100 |
| | Carbopol® Ultrez 20 Polymer | Acrylates/C10-30 alkyl acrylate crosspolymer | 0,30 |
| **B** | Hydrolactol® 70 | Glyceryl stearate (and) Propylene glycol stearate (and) Glyceryl isostearate (and) Propylene glycol isostearate (and) Oleth-25 (and) Ceteth-25 | 2,00 |
| | Glycerin | Glycerin | 2,00 |
| **C** | MinaCare Hexiol | 1,2-Hexanediol | 2,25 |
| | MinaCare Pentiol | Pentylene glycol | 3,00 |
| | MinaCare Hexcine | Ethylhexylglycerin | 2,00 |
| | Tocopherol | Tocopherol | 0,15 |
| D | 10 %ige Natronlauge | Aqua (and) sodium hydroxide | q. sat. |
| E | **Entweder:** Lösung von L-Menthol (40 %) in 1,2-Hexandiol (50 %) und Wasser (10 %) | **Entweder:** 1,2-Hexanediol (and) Menthol (and) Aqua | 1,25 |
| | **Oder:** Lösung von WS-23 (40 %) in 1,2-Hexandiol (50 %) und Wasser (10 %) | **Oder**; 1,2-Hexanediol (and) Menthyl diisopropyl propionamide (and) Aqua | |
| F | Citrat-Puffer, pH 6 | Aqua (and) Citric acid (and) Sodium citrate | 0,15 |

Herstellung: Carbopol® Ultrez 20 Polymer wird bei 20-25 °C in Wasser eingerührt und 5 min nachgerührt (Phase A). Die Komponenten der Phase B werden bei 20-25 °C vermischt. Phase A und Phase B werden separat unter langsamem Rühren (400 U/min) auf 55-60 °C erhitzt. Bei Erreichen der Solltemperatur wird Phase B unter Rühren (1000-1500 U/min) zu Phase A gegeben. Anschließend wird die Mischung 30 min bei 55-60 °C nachgerührt und dann unter Rühren auf 40 °C abgekühlt. Bei dieser Temperatur wird Phase C zugegeben und der pH-Wert wird mit Phase D auf pH 6,0 eingestellt. Nach Abkühlung auf 20-25 °C werden die Phasen E und F unter Rühren zugegeben.

Beide Gele wurden einem sensorischen Effektivitätstest mit 66 Testpersonen im Alter von 19 bis 50 Jahren (50 % Frauen, 50 % Männer) unterzogen. Dabei wurden beide Produkte an jeweils zwei unterschiedlichen Körperstellen appliziert: 1) im Gesicht an der Unteraugen-Partie und 2) am inneren Vorderarm. Drei sensorische Kriterien wurden beurteilt:

| | **WS-23/ 1,2-Hexandiol/ H₂O** | | **L-Menthol/ 1,2-Hexandiol/ H₂O** | |
|---|---|---|---|---|
| | **Unterauge** | **Vorderarm** | **Unterauge** | **Vorderarm** |
| Zeit bis zum Eintreten des Kühleffekts in Sekunden | 16,2 | 27,3 | 7,4 | 16,3 |
| Dauer des Kühleffekts in Minuten | 11,5 | 2,6 | 7,2 | 1,4 |
| Intensität des Kühleffekts, Skala: 0 (sehr schwacher Effekt) bis 10 (sehr starker Effekt) | 7,6 | 8,3 | 3,8 | 4,0 |

Aus den Daten geht hervor, dass mit Hilfe die beiden verschiedenen Kühlreagenzien unterschiedliche sensorische Effekte erzielt werden.

### Beispiel 7 - Klares Aftershave-Gel auf Methylcellulose-Basis

| **Phase** | **Einsatzstoff** | **INCI** | **%** |
|---|---|---|---|
| **A** | Wasser | Aqua | ad |
| | MinaCare Caprocine | Capryloyl glycine | 0,50 |
| **B** | Methocel® MC 40-0202 PCG | Hydroxypropyl methylcellulose | 1,00 |
| | 10 %ige Natronlauge | Aqua (and) sodium hydroxide | q. sat. |
| **C** | Lösung von WS-23 (50 %) in 1,2-Hexandiol (50 %) | Methyl diisopropyl propionamide (and) 1,2-Hexanediol | 1,00 |
| | MinaCare Pentiol | Pentylene glycol | 4,50 |
| | 1,2-Butandiol | 1,2-Butanediol | 8,00 |
| **D** | DL-Milchsäure | Lactic acid | q. sat. |

Herstellung: MinaCare Caprocine wird in Wasser aufgenommen und die Mischung wird auf 70-75 °C erwärmt. Methocel® MC 40-0202 PCG wird in der Wärme zugegeben. Der pH-Wert wird durch Zugabe von Natronlauge auf pH > 8,50 eingestellt. Anschließend wird die Mischung unter langsamem Rühren auf 40 °C abgekühlt. Phase C wird zugegeben und die Mischung wird 15-20 min bei 800 U/min nachgerührt. Abschließend wird der pH-Wert durch Zugabe von DL-Milchsäure auf pH 5,5-6,0 eingestellt.

### Beispiel 8 - Opakes Aftershave-Gel mit Haut aufhellender Wirkung

| **Phase** | **Einsatzstoff** | **INCI** | **%** |
|---|---|---|---|
| **A** | Wasser | Aqua | ad |
| | Carbopol® Ultrez 20 Polymer | Acrylates/C10-30 alkyl acrylate crosspolymer | 0,2 |
| **B** | Lösung von WS-23 (50 %) in 1,2-Hexandiol (50 %) | Methyl diisopropyl propionamide (and) 1,2-Hexanediol | 1,00 |
| | MinaCare Pentiol | Pentylene glycol | 4,50 |
| | 1,2-Propandiol | Propylene glycol | 4,00 |
| | Ellagsäure | Ellagic Acid | 0,5 |
| **C** | Cyclopentasiloxan | Cyclopentasiloxane | 4,00 |
| | MinaCare Hexcine | Ethylhexylglycerin | 1,00 |
| | Tocopherol | Tocopherol | 0,05 |
| | Parfum | Parfum (Fragrance) | 0,10 |
| **D** | 10%ige Natronlauge | Sodium hydroxide | q. sat. |
| **E** | Citrat-Puffer, pH 6 | Aqua (and) Citric acid (and) Sodium citrate | 0,15 |

Herstellung: Carbopol® Ultrez 20 Polymer wird in Wasser suspendiert und 5 min bei 20-25 °C langsam gerührt. Nacheinander werden Phasen B und C unter Rühren (1000-1200 U/min) zugegeben. Abschließend wird der pH-Wert mit Natronlauge auf pH 6,0 eingestellt und durch Zugabe von Citratpuffer stabilisiert.

### Beispiel 9 - Lippenstift mit Kühlwirkung

| **Phase** | **Einsatzstoff** | **INCI** | **%** |
|---|---|---|---|
| **A** | Bienenwachs | Cera alba | 20,00 |
| | Ricinusöl (Castoröl) | Ricinus communis oil | 45,00 |
| | Jojobaöl | Simmondsia chinensis oil | 23,00 |
| | Lipex® Cocoasoft | Theobroma cacao butter | 10,00 |
| | Lösung von WS-23 (50 %) in Ethylhexylglycerin (50 %) | Ethylhexylglycerin (and) Methyl diisopropyl propionamide | 1,00 |
| | Pigmentmischung "Baby Pink" | CI 77891 (and) CI 75470 (and) CI 77019 | 0,70 |
| **B** | Parfum | Parfum | 0,20 |
| | Tocopherol | Tocopherol | 0,10 |

Herstellung: Phase A wird unter Rühren aufgeschmolzen. Bei 80 °C wird Phase B zugegeben. Die Mischung wird rasch in eine Gussform gegossen und anschließend 30 min bei 5 °C gelagert.

### Beispiel 10 - Massageöl mit Kühlwirkung

| **Phase** | **Einsatzstoff** | **INCI** | **%** |
|---|---|---|---|
| **A** | Macadamianuss-Öl | Macadamia ternifolia seed oil | 29,55 |
| | Lipovol® HNO | Corylus avellana nut oil | 7,00 |
| | Lipovol® SES | Sesamum indicum oil | 20,00 |
| | Lipovol® J | Simmondsia chinensis oil | 7,00 |
| | Bergacare® EM-16 | Isopropyl myristate | 30,00 |
| | MinaCare Silfree OE | Dicaprylyl ether | 4,00 |
| | Parfum | Parfum | 0,30 |
| | Lösung von WS-23 (50 %) in Ethylhexylglycerin (50 %) | Ethylhexylglycerin (and) Methyl diisopropyl propionamide | 2,00 |
| | Tocopherol | Tocopherol | 0,15 |

Herstellung: Alle Inhaltsstoffe werden unter langsamem Rühren (z.B. 200 U/min) vermengt.

## Patentansprüche

1. Stabile Lösung, umfassend
a) zu 10-70 Gew.-% ein physiologisches Kühlreagenz
a1) aus der Gruppe der N-alkylierten Carboxamide der allgemeinen Formel (I) 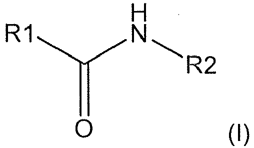 wobei
R1 ein Rest mit 6 bis 12 Kohlenstoffatomen ausgewählt unter verzweigten Alkylresten und Cycloalkyl mit mindestens einem weiteren C1-C3-Alkylsubstituenten am Ring ist; und
R2 ein Rest mit 1 bis 10 Kohlenstoffatomen ausgewählt unter verzweigtem oder unverzweigtem C1-C6-Alkyl, C3-C6-Cycloalkyl und Phenyl ist, wobei R2 einen weiteren Substituenten ausgewählt unter Heteroaryl, -CO₂-C1-C3-Alkyl, -CN, -OH, -H, C1-C3-Alkyl-C(=O)-NH₂ und -O-C1-C3-Alkyl aufweist,
oder
a2) mit cyclischer Monoterpenstruktur der allgemeinen Formel (II) 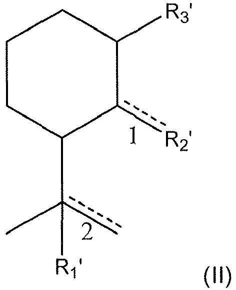 wobei
-----1, 2 jeweils unabhängig voneinander Einfach- oder Doppelbindungen sind
R1' kein Substituent (--- 2 Doppelbindung), Wasserstoff oder -OH (--- 2 Einfachbindung), ist,
R2' -OH (--- 1 Einfachbindung), =O (--- 1 Doppelbindung), -COOH (--- 1 Einfachbindung), -COOCH₂-CH(OH)-CH₂-OH (--- 1 Einfachbindung), -OC(=O)R4', -O-R5' ist oder mit R3' eine Struktur bestehend aus einem oder mehreren kondensierten Ringen bildet, welche mindestens einen weiteren Substituenten -OH und/oder CH₃ trägt,
R3' Wasserstoff ist oder mit R2' die oben beschriebene Struktur aus einem oder mehreren kondensierten Ringen bildet, und
R4',R5' ein C1-C5-Alkyl- oder Cylcoalkylrest ist, welcher mindestens ein weiteres Heteroatom ausgewählt aus Sauerstoff und Stickstoff enthält oder in Form mindestens eines Substituenten aufweist,
und
b) zu 90-30 Gew.-% mindestens einen mindestens zweiwertigen Alkohol gemäß der allgemeinen Formel (III) enthält 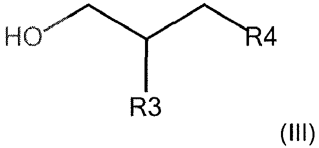 wobei
R3 Wasserstoff oder eine -OH-Gruppe ist und
R4 ausgewählt ist aus -C2-C6-Alkyl, -C2-C6-Alkyl mit OH-Gruppe in variabler Position, -O-C2-C8-Alkyl und -O-C(=O)-C2-C10-Alkyl,
wobei mindestens einer der Reste R3, R4 eine OH-Gruppe ist oder aufweist, und
c) zu 0,1-25 Gew.-% Wasser enthält,
wobei sich die Gewichtsprozente von (a), (b) und (c) zu 100 addieren.

2. Stabile Lösung gemäß Anspruch 1, **dadurch gekennzeichnet, dass** das physiologische Kühlreagenz (a1) ausgewählt ist aus der Gruppe der Substanzen mit R1 gleich verzweigter C1-C6-Alkylrest.

3. Stabile Lösung gemäß einem der Ansprüche 1 und 2, **dadurch gekennzeichnet, dass** das physiologische Kühlreagenz (a1) 2-Isopropyl-N,2,3-trimethylbutanamid (WS-23) ist.

4. Stabile Lösung gemäß einem oder mehreren der Ansprüche 1-3, **dadurch gekennzeichnet, dass** das physiologische Kühlreagenz (a2) gemäß der allgemeinen Formel (II) ausgewählt ist aus Menthol, Mentholestern und -ethern sowie Menthon-Derivaten.

5. Stabile Lösung gemäß Anspruch 4, **dadurch gekennzeichnet, dass** das physiologische Kühlreagenz (a2) gemäß der allgemeinen Formel II L-Menthol ist.

6. Stabile Lösung gemäß einem oder mehreren der Ansprüche 1-5, **dadurch gekennzeichnet, dass** mindestens zwei mindestens zweiwertige Alkohole (b) der allgemeinen Formel (III) vorhanden sind.

7. Stabile Lösung gemäß einem oder mehreren der Ansprüche 1-6, **dadurch gekennzeichnet, dass** der mindestens eine mindestens zweiwertige Alkohol (b) ausgewählt ist aus zwei- oder dreiwertigen Alkoholen mit linearer oder verzweigter C5-C8-Alkylkette.

8. Stabile Lösung gemäß einem oder mehreren der Ansprüche 1-7, **dadurch gekennzeichnet, dass** der mindestens eine mindestens zweiwertige Alkohol (b) ausgewählt ist aus 1,2-Pentandiol, 1,5-Pentandiol, 1,2-Hexandiol, 1,6-Hexandiol, 1,2,3-Hexantriol, Dipropylenglycol, 1,2-Octandiol, Ethylhexylglycerin und Glycerylcaprylat.

9. Verwendung einer stabilen Lösung, gemäß einem oder mehreren der Ansprüche 1-8 für kosmetische, pharmazeutische, dermatologische, hygienische und Lebensmittel Zubereitungen.

10. Kosmetisches, pharmazeutisches, dermatologisches, hygienisches oder Lebensmittel Produkt, enthaltend eine stabile Lösung gemäß einem oder mehreren der Ansprüche 1-8.

## Claims

1. Stable solution, comprising
a) 10-70 % by weight of a physiologic cooling reagent
a1) from the group of N-alkylated carboxamides of the general formula (I) 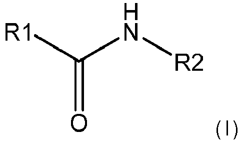 wherein
R1 is a moiety of 6 to 12 carbon atoms selected from branched alkyl moieties and cycloalkyl with at least one further C1-C3 alkyl substituent on the ring; and
R2 is a moiety of 1 to 10 carbon atoms selected from branched or unbranched C1-C6 alkyl, C3-C6 cycloalkyl and phenyl, wherein R2 has a further substituent selected from heteroaryl, -CO₂-C1-C3 alkyl, -CN, -OH, -H, C1-C3 alkyl-C(=O)-NH₂ and -O-C1-C3 alkyl,
or
a2) having a cyclic monoterpene structure of the general formula (II) 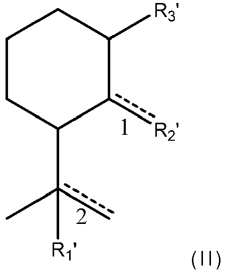
wherein
-----1, 2 are each independently from one another single or double bonds
R1' is not a substituent (--- 2 double bond), hydrogen or -OH (-- 2 single bond),
R2' is -OH (--- 1 single bond), = O (--- 1 double bond), -COOH (-- 1 single bond), -COOCH₂-CH(OH)-CH₂-OH (--- 1 single bond), -OC(=O)R4', -O-R5' or forms a structure with R3' consisting of one or more fused rings, which carries at least one further substituent -OH and/or CH₃,
R3' is hydrogen or forms with R2' the above described structure of one or more fused rings, and
R4', R5' is a C1-C5 alkyl- or cycloalkyl moiety, which contains at least one further heteroatom selected from oxygen and nitrogen or present in form of at least one substituent,
and
b) 90-30 % by weight of at least one at least bivalent alcohol according to the general formula (III) 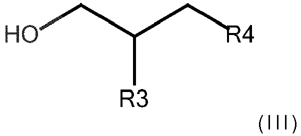 wherein
R3 is hydrogen or an -OH group and
R4 is selected from -C2-C6 alkyl, -C2-C6 alkyl with an OH group at a variable position, -O-C2-C8 alkyl and -O-C(=O)-C2-C10 alkyl,
wherein at least one of the moieties R3, R4 is or has an OH group,
and
c) 0.1-25% by weight water,
wherein the percentages by weight of (a), (b) and (c) add up to 100.

2. Stable solution according to claim 1, **characterized in that** the physiologic cooling reagent (a1) is selected from the group of substances with R1 equal to branched C1-C6 alkyl moiety.

3. Stable solution according to any of claims 1 and 2, **characterized in that** the physiologic cooling reagent (a1) is 2-isopropyl-N,2,3-trimethylbutanamide (WS-23).

4. Stable solution according to one or more of claims 1-3, **characterized in that** the physiologic cooling reagent (a2) according to the general formula (II) is selected from menthol, menthol esters and ethers as well as menthone derivatives.

5. Stable solution according to claim 4, **characterized in that** the physiologic cooling reagent (a2) according to the general formula II is L-menthol.

6. Stable solution according to one or more of claims 1-5, **characterized in that** at least two at least bivalent alcohols (b) of the general formula (III) are present.

7. Stable solution according to one or more of claims 1-6, **characterized in that** the at least one at least bivalent alcohol (b) is selected from bivalent or trivalent alcohols with linear or branched C5-C8 alkyl chain.

8. Stable solution according to one or more of claims 1-7, **characterized in that** the at least one at least bivalent alcohol (b) is selected from 1,2-pentanediol, 1,5-pentanediol, 1,2-hexanediol, 1,6-hexanediol, 1,2,3-hexanetriol, dipropylene glycol, 1,2-octanediol, ethylhexylglycerin and glyceryl caprylate.

9. Use of a stable solution, according to one or more of claims 1-8, for cosmetic, pharmaceutical, dermatological, hygienic and food preparations.

10. Cosmetic, pharmaceutical, dermatological, hygienic or food product, containing a stable solution according to one or more of claims 1-8.

## Revendications

1. Solution stable, comprenant
a) 10 à 70 % en poids d'un réactif de refroidissement physiologique
a1) parmi le groupe des carboxamides N-alkylés de formule générale (I) 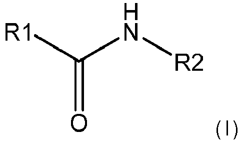 dans laquelle
R1 est un radical ayant 6 à 12 atomes de carbone choisi parmi les radicaux alkyle ramifiés et un cycloalkyle avec au moins un autre substituant alkyle en C1-C3 sur le cycle; et
R2 est un radical ayant 1 à 10 atomes de carbone choisi parmi les groupes alkyle en C1-C6 ramifiés ou non ramifiés, cycloalkyle en C3-C6 et phényle, où R2 présente un autre substituant choisi parmi hétéroaryle, -CO₂-C1-C3 alkyle, -CN, -OH, -H, -H, C1-C3 alkyle-C(=O)-NH₂ et -O-C1-C3 alkyle,
ou
a2) avec une structure monoterpénique cyclique de formule générale (II) 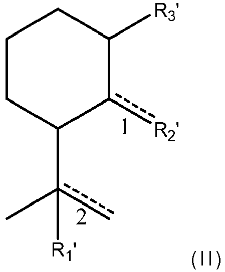 où
-----1, 2 sont chacune indépendamment des liaisons simples ou doubles
R1' n'est pas un substituant (--- 2 liaison double), hydrogène ou -OH (--- 2 liaison simple),
R2' est -OH (--- 1 liaison simple), = O (--- 1 liaison double), -COOH (--- 1 liaison simple), -COOCH₂-CH(OH)-CH₂-OH (--- 1 liaison simple), -OC(=O)R4', -O-R5' ou forme avec R3' une structure constituée d'un ou plusieurs cycles condensés, qui porte au moins un substituant supplémentaire -OH et/ou CH₃,
R3' est l'hydrogène ou forme avec R2' la structure décrite ci-dessus à partir d'un ou plusieurs cycles condensés, et
R4',R5' est un radical alkyle ou cycloalkyle en C1-C5, qui contient au moins un hétéroatome supplémentaire choisi parmi l'oxygène et l'azote ou sous forme présente au moins un substituant,
et
b) 90 à 30 % en poids d'au moins un alcool au moins divalent selon la formule générale (III) 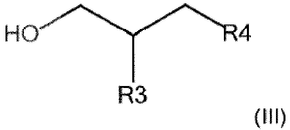 où
R3 est l'hydrogène ou un groupe -OH, et
R4 est choisi parmi l'alkyle en C2-C6, l'alkyle en C2-C6 avec un groupe OH en position variable, -O-alkyle en C2-C8 et -O-C(=O)alkyle en C2-C10,
où au moins l'un des radicaux R3, R4 est ou présente un groupe OH,
et
c) 0,1-25 % en poids d'eau,
où les pourcentages en poids de (a), (b) et (c) totalisent jusqu'à 100.

2. Solution stable selon la revendication 1, **caractérisée en ce que** le réactif de refroidissement physiologique (a1) est choisi parmi le groupe des substances avec R1 identique à un radical alkyle en C1-C6 ramifié.

3. Solution stable selon l'une quelconque des revendications 1 et 2, **caractérisée en ce que** le réactif de refroidissement physiologique (a1) est le 2-isopropyl-N,2,3-triméthylbutanamide (WS-23).

4. Solution stable selon une ou plusieurs des revendications 1-3, **caractérisée en ce que** le réactif de refroidissement physiologique (a2) selon la formule générale (II) est choisi parmi le menthol, les esters et éthers de menthol ainsi que les dérivés de menthone.

5. Solution stable selon la revendication 4, **caractérisée en ce que** le réactif de refroidissement physiologique (a2) selon la formule générale II est le L-menthol.

6. Solution stable selon une ou plusieurs des revendications 1-5, **caractérisée en ce qu'**au moins deux alcools au moins divalents (b) de formule générale (III) sont présents.

7. Solution stable selon une ou plusieurs des revendications 1-6, **caractérisée en ce que** l'au moins un alcool au moins divalent (b) est choisi parmi les alcools divalents ou trivalents ayant une chaîne alkyle linéaire ou ramifiée en C5-C8.

8. Solution stable selon une ou plusieurs des revendications 1-7, **caractérisée en ce que** l'au moins un alcool au moins divalent (b) est choisi parmi le 1,2-pentanediol, le 1,5-pentanediol, le 1,2-hexanediol, le 1,6-hexanediol, le 1,2,3-hexanetriol, le dipropylèneglycol, le 1,2-octanediol, l'éthylhexylglycérine et le caprylate de glycéryle.

9. Utilisation d'une solution stable selon une ou plusieurs des revendications 1-8 pour des préparations cosmétiques, pharmaceutiques, dermatologiques, hygiéniques et alimentaires.

10. Produit cosmétique, pharmaceutique, dermatologique, hygiénique ou alimentaire, contenant une solution stable selon une ou plusieurs des revendications 1-8.
